(19) Europäisches Patentamt
European Patent Office
Office européen des brevets

(11) Publication number: **0 249 352 A1**

(12) # EUROPEAN PATENT APPLICATION

(21) Application number: 87304547.0

(22) Date of filing: 21.05.87

(51) Int. Cl.[4]: C07C 43/04 , C07C 41/06

(30) Priority: 29.05.86 GB 8613080

(43) Date of publication of application: 16.12.87 Bulletin 87/51

(84) Designated Contracting States: AT BE CH DE ES FR GB IT LI NL SE

(71) Applicant: The British Petroleum Company p.l.c.
Britannic House Moor Lane
London EC2Y 9BU(GB)

(72) Inventor: Atkins, Martin Philip
The British Petroleum Company p.l.c.
Chertsey Road
Sunbury-on-Thames Middlesex TW16 7LN(GB)
Inventor: Wren, Ian Michael
The British Petroleum Co. p.l.c. West Britannic
Moor Lane London EC2Y 9BU(GB)

(74) Representative: Richardson, Derek et al
BP INTERNATIONAL LIMITED Patents & Agreements Division Chertsey Road
Sunbury-on-Thames Middlesex TW16 7LN(GB)

(54) **The production of ethers using layered or pillared clay catalysts.**

(57) Ethers are produced by reacting one or more alcohols with one or more alkenes having at least two alkyl substituents on one olefinic carbon atom over an acidic layered or pillared clay catalyst at an olefin:alcohol molar ratio of at least 1:1.

EP 0 249 352 A1

# THE PRODUCTION OF ETHERS USING LAYERED OR PILLARED CLAY CATALYSTS

The invention relates to the production of ethers using layered or pillared clay catalysts.

Both layered and pillared clay catalysts are known and have been proposed for the production of ethers. Thus, the present applicant's European Patent Specifications EP-A-0031252 and EP-A-0031687 disclose and claim processes for the production of ethers by reacting one or more alcohols with one or more olefins in the presence of a cation-exchangeable layered clay, the cation being either a metal or hydrogen. The use of pillared clays in proton catalysed organic reactions is also known.

A layered clay within the context of the present specification is a clay having a lamellar structure with interlamellar spaces disposed between the lamellar layers. Typical of such clays is montmorillonite which has an idealised stoichiometric composition corresponding to $Na_{0\bullet 67}[Al_{3\bullet 33}Mg_{0\bullet 67}](Si_8)O_{20}(OH)_4$. Structurally it comprises a central octahedral co-ordination layer containing aluminium and magnesium oxides and hydroxides sandwiched between two tetrahedral co-ordination layers containing silicon oxide. Normally, in nature, cations are present to compensate for the charge imbalance caused by isomorphous substitution of $Mg^{2+}$ for $Al^{3+}$ in the octahedral layer, and/or $Al^{3+}$ or other ions for $Si^{4+}$ in the tetrahedral layers. The octahedral and tetrahedral regions are tightly bound together to form a lamellar layer. The space between the lamellar layers, i.e. the interlamellar space, is normally occupied by exchangeable $Ca^{2+}$ or $Na^+$ ions. The distance between the interlamellar layers can be substantially increased by absorption of a variety of polar molecules such as water, ethylene glycol, amines etc., which enter the interlamellar space and in doing so push apart the lamellar layers. The interlamellar spaces tend to collapse when the molecules occupying the space are removed, for example by heating the clay at a high temperature. Both natural and synthetic clays having a layered structure are well known and may be used in the process of the invention. Besides montmorillonites such as bentonite and Fullers Earths, other types of suitable clays include hectorites, beidellites, vermiculites and nontronite.

The alkali metal or alkaline earth metal ions in the interlamellar space can be ion-exchanged for hydrogen ions or other metal ions by techniques well known in the art.

The interlamellar spacing of layered clays can be expanded and the tendency of the spacing to collapse at high temperatures can be prevented by inserting props or pillars in the interlamellar space between the lamellar layers. Thus, US Patent No. 4176090 describes and claims an interlayered smectite clay product which includes an inorganic oxide selected from the group consisting of alumina, zirconia and mixtures thereof between the layers thereof, and which possesses an interlayer distance of from about 6 to 16 Angstroms, said interlayered clay having greater than about 50 per cent of its surface area in pores of less than 30 Angstroms in diameter. Such clays can be produced by (a) reacting a smectite with a mixture of a polymeric cationic hydroxy inorganic metal complex selected from the group comprising aluminium and zirconium complexes and mixtures thereof and water to obtain a smectite having greater than 50 per cent of its surface area in pores of less than 30 Angstroms in diameter after dehydration; and (b) separating the interlayered smectite from the mixture. An improvement in this method of preparation is described in US Patent No. 4248739 whereby the smectite is reacted with a high molecular weight cationic hydroxy metal complex and copolymers thereof having a molecular weight of from about 2,00 to 20,000.

The production of ethers is important for the production of individual chemicals and for the production of gasoline blending components. Particular attention has been given to the production of methyl tertiary butyl ether (MTBE) from methanol and an isobutene stream. The catalysts normally used are strongly acidic cation exchange resins consisting essentially of a sulphonated polystyrene resin, for instance, a divinylbenzene cross-linked polystyrene matrix having 0.5 to 20% and preferably 4 to 16% of copolymerised divinylbenzene, attached to which are ionisable or functional nuclear sulphonic acid groups. These resins are manufactured and sold commercially under various trade names. Resins sold by Rohm and Haas under the registered trademark "Amberlyst" are particularly well known as etherification catalysts for MTBE production. The mole ratio of alcohol to olefin is particularly critical in etherification. While a wide range of mole ratios have been mentioned in the literature (including patent specifications), in practice an excess of olefin tends to produce dimerisation or oligomerisation of the olefin. Olefin dimers and oligomers are not as desirable gasoline blending components as ethers and, if the oligomerisation is considerable, it may eventually lead to the undesirable production of gums, particularly with feedstocks containing $C_5$ and higher olefins. The tendency of oligomerisation is particularly pronounced with sulphonated polystyrene resin catalysts so that, in commercial practice, the molar ratio is held at or very near to the theoretical 1:1 molar ratio. An excess of methanol is not of itself harmful but it may require a separate subsequent separation

step either for recycle or because too much methanol in the product may be undesirable. There are practical limits to the amount of methanol that can be included in gasoline blending components because, inter alia, of the sensitivity of the Reid Vapour Pressure of gasoline to relatively low concentrations of methanol and phase separation problems.

An excess of olefin in the MTBE product is not so disadvantageous since it can readily be separated by distillation. The use of an excess of olefin would be desirable therefore, provided a catalyst with low oligomerisation activity could be found.

It has now been found that layered clay catalysts and certain pillared clay catalysts can be as active as sulphonated polystyrene resin catalysts and that they do not show the same tendency to oligomerisation.

According to the present invention, therefore, a process for the production of ethers comprises reacting one or more alcohols with one or more alkenes having at least two alkyl substituents on one olefinic carbon atom over an acidic layered or pillared clay catalyst, characterised in that the olefin: alcohol molar ratio is at least 1:1.

Suitable alcohols include methanol, ethanol, propanols, butanols, pentanols and hexanols.

The alkene reactant is an alkene having at least two alkyl substituents on one olefinic carbon atom. Suitable alkenes have the formula:-

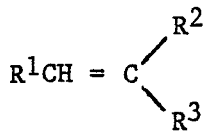

(I)

wherein $R^1$ is either hydrogen, alkyl or cycloalkyl and $R^2$ and $R^3$ are independently alkyl or cycloalkyl groups. In the formula (I), $R^2$ and $R^3$ are preferably independently alkyl groups containing from 1 to 4 carbon atoms and $R^1$ is preferably either hydrogen or an alkyl group containing from 1 to 6, more preferably 1 to 4, carbon atoms. Examples of suitable alkenes having the formula (I) are isobutene, 2-methylbutene-1 and 2-methylbutene-2. Mixtures of alkenes having at least two alkyl substituents on one olefinic carbon atom may be employed. Suitable mixtures include mixtures of alkenes comprising refinery streams. An example of a useful refinery stream is the $C_4$ to $C_6$ olefinic stream derived from light catalytically cracked spirit (LCCS).

A preferred reaction is that of isobutene with methanol to produce methyl tertiary butyl ether (MTBE). Another preferred reaction is that of a methylbutene with methanol to produce a tertiary amyl methyl ether (TAME). A preferred source of monoolefin reactants for use in the process of the present invention is the refinery stream generally referred to as Light Cat Cracked Spirit (LCCS), itself derived from the catalytic cracking of heavier petroleum fractions, normally boiling in the range from 350°C to 550°C, to lighter products. LCCS typically comprises isobutene, n-butane, butene-1, transbut-2-ene, cis but-2-ene, 2-methylbutene-1, n-pentane, isopentane, pent-2-enes and 2-methylbutene-2. A typical process for the production of ethers comprises the steps of:-

(A) reacting LCCS comprising $C_4$ to $C_6$ monoolefins including isobutene and at least one 2-methylbutene, with methanol in the presence of an acidic layered clay or pillared clay catalyst, the molar ratio of monoolefin to methanol being at least 1:1, under conditions of temperature and pressure such as to produce a product principally comprising MTBE, TAME and unreacted $C_4$ to $C_6$ monoolefins, and

(B) separating the product from step (A) into MTBE and TAME and a $C_4$ to $C_6$ monoolefin fraction.

The olefin:alcohol molar ratio is at least 1:1. It is preferably greater than 1:1.

The upper limit of the molar ratio will depend on practical considerations. The excess of olefin should, preferably, be such as to ensure significant conversion of the alcohol. Generally the upper limit of the olefin:alcohol molar ratio is about 5:1. In the production of methyltertiary butyl ether (MTBE) by the reaction of isobutene with methanol this has been found experimentally to occur at about 2:1 molar ratio. Using mixtures of olefins of the formula (I) with olefins which are substantially unreactive under the reaction conditions, for example n-olefins, the olefin:alcohol molar ratio referred to is the molar ratio of the olefin of the formula (I) to the alcohol.

The reduced tendency of the catalysts of the present invention to catalyse oligomerisation may lead on to other advantages. With conversions of alcohol approaching 100% there will be no problems of azeotrope formation in the product, if an aqueous alcohol is used as feedstock. Thus aqueous methanol could be used, the water being removed from the product by simple distillation. Olefin fractions derived from cracking normally contain some dienes and it is recommended that they to be removed before the etherification reaction. The present catalysts may be more tolerant of dienes, thereby avoiding the need for a preliminary diene separation step.

The etherification temperature may be in the range 40 to 120°C and the space velocity (LHSV) from 0.2 to 6.0 $h^{-1}$. The reaction may proceed in either the liquid or vapour phase, preferably the former. The pressure may be from 4 to 80 bars gauge, for liquid phase reaction.

The preferred catalyst is an acidic layered clay, preferably an acidic smectite-type layered clay, more preferably an $H^+$-exchanged smectite-type layered clay. Smectite-type layered clays include montmorillonites, of which bentonites and Fuller's earths are examples, hectorites, beidellites, vermiculites and nontronite. A preferred clay is montmorillonite, particularly a Texas montmorillonite or a Wyoming bentonite.

Substituting a metal cation for the hydrogen cation appears to reduce the activity, although significant activity was noted from an $Al^{3+}$ exchanged montmorillonite. Highly polarising metal cations may,therefore, be alternatives to hydrogen cations.

Al-or Zr-pillared clays have also been found to be less active than hydrogen exchanged layered clays, although the activity of such pillared clays and an $Al^{3+}$ exchanged layered clay can be at least partially restored by silylation or by acid treatment. A suitable silylation process for layered clays is described in EP-A-0150897. A suitable silylation process for pillared clays is described in EP-A-0150898.

Silylation of layered clays tends to reduce activity, so the silylation effect with pillared or Al-exchanged layered clays may be due to the formation of aluminosilicate or increased hydrophobicity of the material.

The invention is illustrated by the following Examples.

Example 1

This Example compares the activity of a number of layered and pillared clays for MTBE production at an isobutene/olefin ratio of 1:1.

The apparatus used was a continuous flow unit having a 25 ml reactor immersed in an oil both to achieve uniform heat transfer. The reactor contained 10 ml of 0.5-1 mm size catalyst particles. Isobutene and methanol at a 1:1 molar ratio were passed over the catalyst at an LHSV of 4 v/v/hr. The isobutene/methanol feed was diluted with pentane (80%). The reactor was maintained at 80°C and 15 bars, giving liquid phase operation. Product from the reactor was cooled to 0°C and analysed by gas chromatography. Each run was of 8 hours duration.

Table 1 below gives the MTBE production for a variety of clay catalysts. A number of alternative comparative catalysts were alsotested, the results with these also being given in Table 1.

TABLE 1

| Catalyst | % wt MTBE in Product Stream |
|---|---|
| $H^+$ montmorillonite (Wyoming) | 10.0 |
| $H^+$ montmorillonite (Texas) | 8.5 |
| $Al^{3+}$ montmorillonite (Wyoming) | 7.5 |
| Silylated $Al^{3+}$ montmorillonite (Wyoming) | 10.0 |
| Silylated $H^+$ montmorillonite (Wyoming) | 7.0 |
| Sulphonated polystyrene resin (Amberlyst 15) | 9.4 |
| H-ZSM 5 zeolite | 4.0 |
| Phosphoric acid on keiselguhr | 0.2 |

TABLE 2

| Feed Ratio: Methanol:Isobutene (molar) | 0.6:1 | 0.8:1 | 1.0:1 | 1.5:1 |
|---|---|---|---|---|
| Component (by GC/MS Analysis) | | | | |
| Dimethyl ether (% wt) | | | | |
| $H^+$-montmorillonite (Example 3) | 0.003 | 0.008 | 0.009 | 0.01 |
| Amberlyst 15 (CT 2) | 0.07 | 0.07 | 0.14 | 0.28 |
| MTBE (% wt) | | | | |
| $H^+$-montmorillonite (Example 3) | 9.2 | 13.1 | 15.0 | 14.0 |
| Amberlyst 15 | 7.3* | 11.8 | 18.0 | 15.0 |

*Significant production (ca. 1% wt) of methyl secbutyl ether observed.

The results summarised in Table 2 demonstrate that the $H^+$-montmorillonite produces significantly lower levels of dimethyl ether than Amberlyst 15 over the entire range of feedstock compositions tested. The differences in dimethyl ether formation with the $H^+$-montmorillonite and Amberlyst 15 was most pronounced at the high methanol:isobutene feed levels (1.5:1) typically employed in commercial MTBE units to maximum isobutene conversion per pass, the clay producing 100 ppm dimethyl ether compared with 2800 ppm for the Amberlyst 15 resin. It is our experience that better (more selective) performance is obtainable using upward flow mode of operation and hence further improvements in dimethyl ether production should be attainable.

The results on dimethyl ether production are significant because straight-chain ethers, e.g. DME, are not as useful as gasoline components as branched-chain ethers, e.g. MTBE.

In addition to the above data, it was observed that under methanol lean operation (methanol:isobutene = ca. 0.6:1 molar) the Amberlyst 15 resin gave a four fold increase in selectivity to undesirable $C_8$ dimers as compared with the $H^+$-montmorillonite.

## Claims

1 A process for the production of ethers by reacting one or more alcohols with one or more alkenes having at least two alkyl substituents on one olefinic carbon atom over an acidic layered or pillared clay catalyst, characterised in that the olefin:alcohol molar ratio is at least 1:1.

2 A process according to claim 1 wherein the olefin:alcohol molar ratio is greater than 1:1.

3 A process according to either claim 1 or claim 2 wherein the alcohol is either methanol, ethanol, a propanol, a butanol, a pentanol or a hexanol.

4 A process according to any one of the preceding claims wherein the alkene has the formula:-

$$R^1CH = C\begin{matrix} / R^2 \\ \backslash R^3 \end{matrix} \qquad (I)$$

wherein $R^1$ is either hydrogen, alkyl or cycloalkyl and $R^2$ and $R^3$ are independently alkyl or cycloalkyl groups.

5 A process according to claim 4 wherein the alkene is one or more of isobutene, 2-methylbutene-1 or 2-methylbutene-2.

6 A process according to any one of the preceding claims wherein methanol is reacted with isobutene to produce methyl tertiary butyl ether.

7 A process according to claim 6 wherein the isobutene:methanol molar ratio is about 2:1.

8 A process according to any one of claims 1 to 5 wherein a methylbutene is reacted with methanol to produce a tertiary amyl methyl ether.

9 A process according to any one of the preceding claims wherein the catalyst is an $H^+$-exchanged smectite-type layered clay.

10 A process according to claim 9 wherein the smectite-type layered clay is montmorillonite.

11 A process for the production of ethers comprises the steps of:

(A) reacting LCCS comprising $C_4$ to $C_6$ monoolefins, including isobutene and at least one 2-methylbutene, with methanol in the presence of an acidic layered clay or pillared clay catalyst, the molar ratio of monoolefin to methanol being at least 1:1, under conditions of temperature and pressure such as to produce a product principally comprising MTBE, TAME and unreacted $C_4$ to $C_6$ monoolefins, and

(B) separating the product from step (A) into MTBE and TAME and a $C_4$ to $C_6$ monoolefin fraction.

Table 1 shows that a hydrogen montmorillonite layered clay had the same activity as a sulphonated polystyrene resin catalyst and considerably more than that of a hydrogen zeolite or supported phosphoric acid.

Montmorillonite exchanged with a highly polarising $Al^{3+}$ cation also showed significant activity although lowly polarising cations gave poor results (0.1% MTBE production for a $Cu^{2+}$ exchanged montmorillonite and 0.2% MTBE production for a $Mg^{2+}$ exchanged montmorillonite).

Silylating the $Al^{3+}$ exchanged montmorillonite increased the activity to that of the $H^+$ montmorillonite, despite the fact that silylating the $H^+$ montmorillonite itself reduced its activity.

Example 2

The procedure of Example 1 was repeated using an $H^+$ montmorillonite as catalyst under the following conditions:

Feed: 2.3:1 molar isobutene:methanol
Temperature: 80°C
Residence Time: 15 minutes (LHSV = $4h^{-1}$)
Pressure: 15 bar
Essentially complete conversion of methanol to MTBE was obtained.
The ratio of dimers:MTBE was 0.7:1.

Comparison Test 1

Example 2 was repeated except that a sulphonated polystyrene resin (Amberlyst 15) was employed as catalyst in place of $H^+$ montmorillonite.

Essentially complete conversion of methanol to MTBE was obtained.

The ratio of dimers to MTBE was 2.3:1.

This Test and Example 2 demonstrate that the clay catalyst produces less undesirable isobutene dimers than the resin catalyst.

Example 3 - Larger Scale Operation

A 75 ml capacity reactor charged with $H^+$-montmorillonite was set up for a trickle flow mode of operation (downward flow) for the production of MTBE from a $C_4$ raffinate stream and methanol. The reactor was operated under typical MTBE production conditions (15 bar, 75°C and LHSV = $2h^{-1}$).

The product was analysed for MTBE and dimethyl ether by GC/MS analysis at different methanol:isobutene molar ratios, i.e. 0.6:1, 0.8:1, 1.0:1 and 1.5:1.

The results are given in Table 2.

Comparison Test 2

Example 2 was repeated except that the reactor was charged with Amberlyst 15 instead of $H^+$-montmorillonite.

The results are given in Table 2.

European Patent Office

# EUROPEAN SEARCH REPORT

Application number

EP 87 30 4547

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | EP-A-0 150 897 (BRITISH PETROLEUM)<br>* Examples 4,5 *<br>--- | 1,3-6, 9 | C 07 C 43/04<br>C 07 C 41/06 |
| A,D | EP-A-0 031 687 (BRITISH PETROLEUM)<br>* Page 2, lines 5-15; page 6, lines 10-25; examples 13-16 *<br>--- | 1-11 | |
| A | EP-A-0 083 970 (BRITISH PETROLEUM)<br>* Page 2, line 25 - page 4, line 33; example 9 *<br>----- | 1-11 | |
| | | | **TECHNICAL FIELDS SEARCHED (Int. Cl.4)** |
| | | | C 07 C 41/00<br>C 07 C 43/00 |

The present search report has been drawn up for all claims

| Place of search | Date of completion of the search | Examiner |
|---|---|---|
| THE HAGUE | 23-09-1987 | WRIGHT M.W. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82